# EUROPÄISCHE PATENTSCHRIFT

(11) **EP 3 733 313 B1**
(45) Veröffentlichungstag und Bekanntmachung des Hinweises auf die Patenterteilung: **17.04.2024**
(21) Anmeldenummer: 20171991.1
(22) Anmeldetag: 29.04.2020
(51) Int. Cl.: B06B 3/00

(54) **LITHOTRIPSIEVORRICHTUNG UND TESTVERFAHREN ZUM BETRIEB EINER LITHOTRIPSIEVORRICHTUNG**
LITHOTRIPSY DEVICE AND TEST METHOD FOR OPERATING A LITHOTRIPSY DEVICE
DISPOSITIF DE LITHOTRIPSIE ET PROCÉDÉ D'ESSAI DESTINÉ AU FONCTIONNEMENT D'UN DISPOSITIF DE LITHOTRIPSIE

(30) Priorität: 30.04.2019 DE 102019111100
(43) Veröffentlichungstag der Anmeldung: 04.11.2020
(73) Patentinhaber: Karl Storz SE & Co. KG, 78532 Baden-Württemberg Tuttlingen (DE)
(72) Erfinder: Göbel, Werner, 78532 Tuttlingen (DE); Hinding, Thomas, 78532 Tuttlingen (DE); Krattiger, Beat, 78532 Tuttlingen (DE); Glöggler, Bernhard, 78532 Tuttlingen (DE)

(56) Entgegenhaltungen:
- EP-A1- 1 163 883
- EP-A1- 2 529 678
- DE-A1-102019 128 043
- US-A1- 2004 127 925
- US-A1- 2010 256 536
- Anonymous: "| MedWOW's news Extracorporeal Shockwave Lithotripsy Defined", , 1. November 2011 (2011-11-01), XP055730106, Gefunden im Internet: URL:https://web.archive.org/web/2011110120 1854/http://www.medwow.com/articles/lithot ripter/extracorporeal-shockwave-lithotrips y-defined/ [gefunden am 2020-09-11]

## Beschreibung

### Stand der Technik

Die Erfindung betrifft eine Lithotripsievorrichtung nach Anspruch 1, einen Lithotripter mit einer Lithotripsievorrichtung nach Anspruch 16 sowie ein Verfahren zum extrakorporalen Testbetrieb einer Lithotripsievorrichtung nach Anspruch 17.

Aus der US 6,875,220 B2 ist bereits eine Lithotripsievorrichtung bekannt, welche eine Sonotrode, wenigstens einen Ultraschallgenerator und wenigstens ein Ultraschallhorn sowie ein Projektil umfasst, wobei das Projektil in wenigstens einem Betriebszustand die Sonotrode zumindest mittelbar beaufschlagt.

Die EP 1 163 883 A1 beschreibt eine Lithotripsievorrichtung, welche zwischen einer elektrisch angesteuerten Schwingungsanregung einer Metallsonde mittels eines Ultraschallwandlers und einer Stoß- und/oder Druckwelle auf die Metallsonde mittels eines reversibel angetriebenen Schlagteils umschaltbar ist. Das Schlagteil ist hydraulisch, elektrisch oder pneumatisch mittels eines proximalseitigen Druckanschlusses antreibbar.

US 2004/127925 A1 offenbart ein Instrument für die Lithotripsie, bei welchem eine feste Sonotrode am distalen Ende eines Horns mittels eines Formstücks zur Schwingungsanregung mittels eines Ultraschallaktivators befestigt ist, wobei um das Formstück eine freie Masse angeordnet ist. Die freie Masse oszilliert bei Anregung mittels des Ultraschallaktivators zwischen dem distalen Ende des Horns und einem Kragen einer schwimmenden Sonotrode, welche konzentrisch außen um die feste Sonotrode gleitbar gelagert ist.

Die Aufgabe der Erfindung besteht insbesondere darin, eine gattungsgemäße Vorrichtung mit verbesserten Eigenschaften hinsichtlich einer Effizienz, insbesondere in Hinblick auf einen Bauraum und/oder eine Leistung, bereitzustellen. Die Aufgabe wird erfindungsgemäß durch die Merkmale des Patentanspruchs 1 gelöst, während vorteilhafte Ausgestaltungen und Weiterbildungen der Erfindung den Unteransprüchen entnommen werden können.

### Vorteile der Erfindung

Die Erfindung geht aus von einer Lithotripsievorrichtung, insbesondere einer intrakorporalen Lithotripsievorrichtung, mit wenigstens einer Ultraschalleinheit, welche wenigstens eine Sonotrode, wenigstens einen Ultraschallgenerator, der dazu ausgebildet ist Ultraschallwellen bereitzustellen, und wenigstens ein Ultraschallhorn, das zur Fokussierung und zumindest mittelbaren Übertragung der Ultraschallwellen von dem Ultraschallgenerator auf die Sonotrode ausgebildet ist, umfasst, und mit wenigstens einer Projektileinheit, welche wenigstens ein linear beweglich gelagertes Projektil umfasst, das dazu ausgebildet ist, in wenigstens einem Betriebszustand die Sonotrode zumindest mittelbar zu beaufschlagen, und das in dem Betriebszustand zumindest mittelbar von dem Ultraschallgenerator mittels bereitgestellter Ultraschallwellen zu einer Bewegung angeregt wird.

Es wird vorgeschlagen, dass das Projektil im Bereich des Ultraschallhorns beweglich gelagert angeordnet ist, wobei das Projektil das Ultraschallhorn koaxial umgebend oder innerhalb eines von dem Ultraschallhorn eingeschlossenen Volumens angeordnet ist.

Hierdurch kann insbesondere eine Effizienz verbessert werden. Vorteilhaft kann ein Bauraum verringert werden. Weiter vorteilhaft kann eine Leistung zur Zertrümmerung von Konkrement verbessert werden.

Unter einer "Lithotripsievorrichtung" soll insbesondere ein, vorzugsweise funktionsfähiger Bestandteil, insbesondere eine Unterbaugruppe und/oder eine Konstruktions- und/oder eine Funktionskomponente eines Lithotripters verstanden werden. Vorzugsweise kann die Lithotripsievorrichtung den Lithotripter zumindest teilweise, vorzugsweise zumindest zu einem Großteil und besonders bevorzugt vollständig ausbilden. Die Lithotripsievorrichtung ist zu einer Zertrümmerung von Konkrement ausgebildet. Unter "Konkrementen" sollen insbesondere sich in Körpern durch Abscheidung entstehende feste Gebilde verstanden werden, welche zumindest teilweise, vorzugsweise zumindest zu einem Großteil und besonders bevorzugt vollständig aus Salzen bestehen, wie beispielsweise Nierensteinen, Gallensteinen, Harnsteinen, Speichelsteinen oder dergleichen. Die Lithotripsievorrichtung ist vorzugsweise als eine intrakorporale Lithotripsievorrichtung ausgebildet, welche dazu ausgebildet ist, zumindest teilweise und vorzugsweise zumindest zu einem Großteil in eine insbesondere künstliche und/oder natürliche Öffnung, insbesondere Körperöffnung, eingeführt zu werden, um sich dort angesammeltes Konkrement zu zertrümmern und/oder zu entfernen. Unter "ausgebildet" soll insbesondere speziell programmiert, eingerichtet, ausgelegt und/oder ausgestattet verstanden werden. Darunter, dass ein Objekt zu einer bestimmten Funktion ausgebildet ist, soll insbesondere verstanden werden, dass das Objekt diese bestimmte Funktion in zumindest einem Anwendungs- und/oder Betriebszustand erfüllt und/oder ausführt. Unter dem Ausdruck "zumindest zu einem Großteil" soll dabei insbesondere zumindest zu 55 %, vorzugsweiser zumindest zu 65 %, bevorzugt zumindest zu 75 %, besonders bevorzugt zumindest zu 85 % und ganz besonders bevorzugt zumindest zu 95 % sowie vorteilhaft vollständig verstanden werden und zwar insbesondere mit Bezug auf ein Volumen und/oder eine Masse eines Objekts. Der Lithotripter ist vorzugsweise Teil eines Lithotripsiesystems, welches zudem noch vorzugsweise zumindest ein Steuergerät und/oder wenigstens eine Fluidpumpe umfasst, welche mit dem Lithotripter verbunden sind/ist.

Unter einer "Ultraschalleinheit" soll insbesondere eine Einheit verstanden werden, welche dazu ausgebildet ist, Ultraschallwellen zu erzeugen, bereitzustellen und/oder an ein zu zertrümmerndes Konkrement zu leiten sowie damit zu beaufschlagen. Unter "Ultraschallwellen" sollen insbesondere Schallwellen oberhalb eines Hörfrequenzbereichs des Menschen verstanden werden. Im vorliegenden Fall weist die Ultraschallschwingung insbesondere eine Ultraschallfrequenz von zumindest 10 kHz, vorzugsweise zumindest 15 kHz und besonders bevorzugt zumindest 20 kHz und/oder von höchstens 100 kHz, vorzugsweise von höchstens 80 kHz und besonders bevorzugt von höchstens 56 kHz auf. Ganz besonders bevorzugt beträgt die Ultraschallfrequenz zwischen 22 kHz und 55 kHz Der Ultraschallgenerator ist insbesondere dabei dazu ausgebildet, Ultraschallwellen zu erzeugen und vorzugsweise weitere Komponenten der Ultraschalleinheit bereitzustellen. Der Ultraschallgenerator weist insbesondere zumindest einen, vorzugsweise zumindest zwei, besonders bevorzugt zumindest drei und ganz besonders bevorzugt eine Vielzahl von Ultraschallaktoren auf, welcher/welche insbesondere als ein Piezoaktor/Piezoaktoren ausgebildet ist/sind. Insbesondere für den Fall, dass der Ultraschallgenerator zumindest zwei Ultraschallaktoren umfasst liegen diese zumindest mittelbar aneinander an, um vorteilhaft eine Intensität des erzeugten Ultraschalls zu erhöhen. Der Ultraschallgenerator ist insbesondere mit dem Ultraschallhorn wirkverbunden.

Insbesondere um den Ultraschall zu übertragen und zu fokussieren weist das Ultraschallhorn vorzugsweise eine sich vorzugsweise konkav verjüngende Zylinderform auf. Vorzugsweise besteht das Ultraschallhorn zumindest teilweise, vorzugsweise zumindest zu einem Großteil und besonders bevorzugt vollständig aus Metall, wie beispielsweise Eisen, Titan, Stahl oder einer Metalllegierung.

Das Ultraschallhorn ist insbesondere mit der Sonotrode wirkverbunden. Vorzugsweise weist die Ultraschalleinheit eine Verbindungseinheit auf, welche zu einer kraft- und/oder formschlüssigen Verbindung der Sonotrode und des Ultraschallhorns ausgebildet ist. Unter einer "kraft-und/oder formschlüssigen Verbindung" ist insbesondere zu verstehen, verbunden, vorzugsweise lösbar verbunden zu sein, wobei eine Haltekraft zwischen zwei Objekten vorzugsweise über einen geometrischen Eingriff der Strukturkomponenten ineinander und/oder über eine Reibungskraft, die vorzugsweise zwischen den Objekten wirkt, übertragen wird. Vorzugsweise handelt es sich bei der Verbindung, um eine Flanschverbindung. Ganz besonders bevorzugt handelt es sich bei der Verbindung um eine Schraubverbindung mittels eines Gewindes. Ferner könnte die Verbindungseinheit zu einer stoffschlüssigen Verbindung ausgebildet sein, welche die Sonotrode und das Ultraschallhorn stoffschlüssig verbindet. Unter "stoffschlüssig verbunden" soll insbesondere verstanden werden, dass die Objekte durch atomare oder molekulare Kräfte zusammengehalten werden, wie beispielsweise beim Löten, Schweißen, Kleben und/oder Vulkanisieren. Ferner ist denkbar, dass das Ultraschallhorn und die Sonotrode einstückig miteinander ausgebildet/verbunden sind. Darunter, dass "ein Objekt und ein weiteres Objekt zumindest teilweise einteilig ausgebildet/verbunden sind", soll insbesondere verstanden werden, dass zumindest ein Element und/oder Teil des Objekts und zumindest ein Element und/oder Teil des weiteren Objekts einteilig ausgebildet/verbunden sind. Unter "einstückig" soll insbesondere zumindest stoffschlüssig verbunden, beispielsweise durch einen Schweißprozess, einen Klebeprozess, einen Anspritzprozess und/oder einen anderen, dem Fachmann als sinnvoll erscheinenden Prozess, und/oder vorteilhaft in einem Stück geformt verstanden werden, wie beispielsweise durch eine Herstellung aus einem Guss und/oder durch eine Herstellung in einem Ein- oder Mehrkomponentenspritzverfahren und vorteilhaft aus einem einzelnen Rohling. Die Ultraschallsonde weist insbesondere eine Form eines Rohrs auf. Die Ultraschallsonde ist dazu ausgebildet bei einem Lithotripsieverfahren das Konkrement zumindest mittelbar zu kontaktieren. Ferner ist die Ultraschallsonde dazu ausgebildet, in eine Körperöffnung eingeführt zu werden. Unter "zumindest mittelbar" soll insbesondere mittelbar aber auch unmittelbar verstanden werden. Die Ultraschallsonde besteht insbesondere zumindest teilweise, vorzugsweise zumindest zu einem Großteil und besonders bevorzugt vollständig aus Metall, wie beispielsweise Eisen, Titan, Stahl oder einer Metalllegierung.

Unter einer "Projektileinheit" soll insbesondere eine Einheit verstanden werden, welche dazu vorgesehen ist, die Sonotrode mit wenigstens einem, vorzugsweise mehreren Stößen zu beaufschlagen. Insbesondere ist das Projektil dazu ausgebildet bei einer Bewegung die Sonotrode mit einer Stoßfrequenz zu beaufschlagen, welche wesentlich kleiner ist als die Ultraschallfrequenz. Im vorliegenden Fall soll unter "wesentlich kleiner" zumindest um einen Faktor 10, vorzugsweise zumindest um einen Faktor 100 und besonders bevorzugt zumindest um einen Faktor 1000 geringer verstanden werden. Das Projektil weist insbesondere eine Form eines Zylinders, insbesondere Hohlzylinders auf. Vorzugsweise besteht das Projektil zumindest teilweise aus Metall, wie beispielsweise Stahl, insbesondere gehärteter Stahl oder Federstahl. Besonders bevorzugt weist das Projektil eine Beschichtung auf, wie insbesondere eine Härtungsbeschichtung, Gleitbeschichtung, eine Antihaftbeschichtung, eine Korrosionsbeschichtung oder dergleichen. Vorzugsweise ist die Antihaft-, Gleit, und/oder Korrosionsbeschichtung auf in einem Betriebszustand an weiteren Flächen reibenden Flächenabschnitten, wie beispielsweise einer Mantelfläche, des Projektils angeordnet. Bei der Antihaft-, Gleit, und/oder Korrosionsbeschichtung kann es sich beispielsweise um eine PTFE-, Keramik-, Kohlenstoff-Beschichtung oder dergleichen handeln. Vorzugsweise ist die Härtungsbeschichtung auf in einem Betriebszustand auf weitere Flächen stoßenden Flächenabschnitten, wie beispielsweise einer Deckfläche, des Projektils angeordnet. Bei der Härtungsbeschichtung kann es sich beispielsweise um eine Metall-Legierung, insbesondere Titanlegierung handeln. Darunter, dass das Projektil "im Bereich des Ultraschallhorns angeordnet ist", soll verstanden werden, dass das Projektil das Ultraschallhorn koaxial umgebend oder innerhalb eines von dem Ultraschallhorn eingeschlossenen Volumen angeordnet ist.

Ferner weist die Lithotripsievorrichtung wenigstens eine Fluideinheit auf. Die Fluideinheit ist insbesondere nicht zu einem Antrieb des Projektils ausgebildet. Die Fluideinheit ist insbesondere dazu ausgebildet, ein Fluid zum Spülen und/oder Absaugen bereitzustellen. Bei dem Fluid handelt es sich insbesondere um eine Flüssigkeit, wie beispielsweise Wasser. Die Fluideinheit weist insbesondere wenigstens einen Fluidkanal auf, welcher sich vorzugsweise von einem proximalen Abschnitt bis zu einem distalen Abschnitt der Lithotripsievorrichtung erstreckt. Unter "proximal" soll insbesondere bei einer Bedienung einem Patienten abgewandt und/oder einem Bediener zugewandt verstanden werden. Unter "distal" soll insbesondere bei einer Bedienung einem Patienten zugewandt und/oder einem Bediener abgewandt verstanden werden. Der distale Abschnitt der Lithotripsievorrichtung ist insbesondere dazu ausgebildet, bei einem Betrieb der Lithotripsievorrichtung in eine Körperöffnung eines Patienten eingeführt zu werden. Der proximale Abschnitt der Lithotripsievorrichtung ist insbesondere dazu ausgebildet bei einem Betrieb der Lithotripsievorrichtung außerhalb eines Körpers eines Patienten angeordnet. Eine Haupterstreckung der Lithotripsievorrichtung setzt sich insbesondere zusammen aus der Haupterstreckung des distalen Abschnitts und des proximalen Abschnitts. Unter einer "Haupterstreckung" soll ein Objekt dabei insbesondere eine längste Erstreckung des Objekts entlang deren Haupterstreckungsrichtung verstanden werden. Unter einer "Haupterstreckungsrichtung" eines Objekts soll dabei insbesondere eine Richtung verstanden werden, welche parallel zu einer längsten Kante eines kleinsten gedachten Quaders verläuft, welcher das Objekt gerade noch vollständig umschließt. Unter einem "Fluidkanal" soll insbesondere eine Baueinheit verstanden werden, die zumindest teilweise zu einer Führung eines Fluidstroms vorgesehen ist, und die insbesondere den Fluidstrom, insbesondere zumindest im Wesentlichen senkrecht zu einer Hauptfluidstromrichtung begrenzt und/oder unmittelbar zumindest teilweise, vorzugsweise auf wenigstens drei Seiten, besonders bevorzugt vollständig, vorteilhaft von genau vier Seiten und besonders bevorzugt zumindest entlang einer Umfangsrichtung, vollständig umschließt. Unter einer "Hauptfluidstromrichtung" soll insbesondere eine effektive Strömungsrichtung eines Fluids, vorzugsweise eine über einen Bereich gemittelte Fließrichtung eines Fluids, verstanden werden. Die Fluideinheit weist insbesondere wenigstens einen Fluidanschluss auf, welcher vorzugsweise am freien Ende des proximalen Abschnitts der Lithotripsievorrichtung angeordnet ist. Insbesondere ist die Fluideinheit vorzugsweise über den Fluidanschluss mit einer Fluidpumpe fluidtechnisch verbunden.

Es wird ferner vorgeschlagen, dass die Projektileinheit wenigstens einen Führungskanal umfasst, welcher zumindest zur beweglichen Lagerung des Projektils ausgebildet ist. Es kann vorteilhaft ein Bauraum verringert werden, da die Anordnung des Projektils innerhalb der Projektileinheit erfolgen kann. Der Führungskanal bildet insbesondere ein Lager, vorzugsweise ein Linearlager, für das Projektil aus, wobei es sich bei dem Lager insbesondere um ein Gleitlager handelt. Alternativ könnte das Lager auch ein Wälzlager ausbilden. Vorzugsweise weist der Führungskanal eine Antihaft-, Gleit, und/oder Korrosionsbeschichtung an Flächen auf, welche in einem Betriebszustand an weiteren Flächen des Projektils reiben, wie beispielsweise einer Mantelfläche des Führungskanals. Bei der Antihaft-, Gleit, und/oder Korrosionsbeschichtung kann es sich beispielsweise um eine PTFE-, Keramik-, Kohlenstoff-Beschichtung oder dergleichen handeln. Der Führungskanal ist insbesondere als ein länglicher Kanal ausgebildet. Unter einem "länglichen Bauteil" soll insbesondere ein Bauteil verstanden werden, dessen Haupterstreckung zumindest um einen Faktor fünf, vorzugsweise zumindest um einen Faktor zehn und besonders bevorzugt zumindest um einen Faktor zwanzig größer ist als eine Erstreckung des Bauteils senkrecht zur Haupterstreckungsrichtung, also insbesondere einem Durchmesser des Bauteils. Insbesondere ist ein Außendurchmesser des Projektils kleiner als ein Innendurchmesser des Führungskanals und zwar insbesondere um zumindest 0,5 %, vorzugsweise um zumindest 1 % und besonders bevorzugt um zumindest 5 % und/oder um höchstens 20 % vorzugsweise um höchstens 15 % und besonders bevorzugt höchstens 10 %. Eine Haupterstreckung des Projektils ist insbesondere kleiner als eine Haupterstreckung des Führungskanals und zwar insbesondere um zumindest 10 %, vorzugsweise um zumindest 50 % und besonders bevorzugt um zumindest 100 %.

Weiterhin wird vorgeschlagen, dass die Ultraschalleinheit zumindest teilweise, vorzugsweise zumindest zu einem Großteil und besonders bevorzugt vollständig, den Führungskanal ausbildet. Es kann vorteilhaft ein Bauraum weiter verringert werden. Weiter vorteilhaft kann auf weitere Bauteile zur Lagerung des Projektils verzichtet werden. Der Führungskanal liegt vorzugsweise zumindest teilweise, vorzugsweise zumindest zu einem Großteil und besonders bevorzugt vollständig im proximalen Abschnitt der Lithotripsievorrichtung. Der Führungskanal weist insbesondere eine Haupterstreckung auf, welche gleich oder größer einer Haupterstreckung der Ultraschalleinheit ist. Insbesondere kann der Führungskanal zumindest teilweise, vorzugsweise zumindest zu einem Großteil und besonders bevorzugt vollständig von dem Ultraschallgenerator und/oder der Sonotrode ausgebildet sein. Besonders bevorzugt ist der Führungskanal jedoch zumindest teilweise, vorzugsweise zumindest zu einem Großteil und besonders bevorzugt vollständig von dem Ultraschallhorn ausgebildet. Um eine Konstruktion zu vereinfachen wird deshalb ganz besonders bevorzugt vorgeschlagen, dass das Ultraschallhorn wenigstens einen Führungsabschnitt aufweist, welcher zumindest teilweise, vorzugsweise zumindest zu einem Großteil und besonders bevorzugt vollständig, den Führungskanal ausbildet. Insbesondere ist eine Haupterstreckung des Führungsabschnitts kleiner oder gleich einer Haupterstreckung des Führungskanals. Der Führungsabschnitt ist von wenigstens einer Ausnehmung und zwar insbesondere einer Vollausnehmung des Ultraschallhorns, welche vorzugsweise entlang einer Rotationssymmetrieachse des Ultraschallhorns verläuft, ausgebildet.

Der Führungskanal könnte separat von dem Fluidkanal ausgebildet sein oder den Fluidkanal begrenzen. Um insbesondere Bauteile einzusparen und eine besonders kompakte Bauform zu erzielen wird vorgeschlagen, dass der Führungskanal zumindest teilweise, vorzugsweise zumindest zu einem Großteil, einen zu einer Fluidleitung ausgebildeten Fluidkanal, insbesondere den vorbenannten Fluidkanal der Fluideinheit, ausbildet. Insbesondere bildet der Führungskanal den im proximalen Abschnitt gelegenen Teil des Fluidkanals zumindest teilweise, vorzugsweise zumindest zu einem Großteil und besonders bevorzugt vollständig aus. Der Fluidkanal ist insbesondere ferner zumindest teilweise von der Sonotrode und zwar insbesondere einem Rohr der Sonotrode ausgebildet. Die Sonotrode bildet insbesondere im distalen Abschnitt gelegenen Teil des Fluidkanals zumindest teilweise, vorzugsweise zumindest zu einem Großteil und besonders bevorzugt vollständig aus. Insbesondere verbindet die Verbindungseinheit die Sonotrode und das Ultraschallhorn fluiddicht miteinander.

Das Projektil könnte außerhalb des Fluidkanals angeordnet sein, wobei das Projektil vorzugsweise den Fluidkanal in Umfangrichtung umgebend angeordnet ist. Um vorteilhaft bei einer kompakten Anordnung des Projektils in einem den Fluidkanal ausbildenden Führungskanal einen Fluiddurchfluss nur unwesentlich zu beeinflussen, wird insbesondere vorgeschlagen, dass das Projektil wenigstens einen Fluiddurchlass aufweist. Das Projektil weist insbesondere eine Ausnehmung, insbesondere eine Vollausnehmung auf, welche den Fluiddurchlass ausbildet und welche vorzugsweise entlang einer Rotationssymmetrieachse des Projektils verläuft.

Es wird vorgeschlagen, dass das Projektil in dem Betriebszustand zumindest mittelbar, insbesondere über das Ultraschallhorn, die Verbindungseinheit und/oder die Sonotrode, von dem Ultraschallgenerator zu einer Bewegung angeregt wird. Es kann vorteilhaft ein Bauraum verringert werden, da insbesondere auf eine weitere Aktorik, welche vorzugsweise ausschließlich zur Anregung des Projektils ausgebildet wäre, verzichtet werden, wodurch vorteilhaft ein Bauraum sowie eine Bauteilkomplexität verringert werden kann. Alternativ oder zusätzlich könnte die Lithotripsievorrichtung auch über eine weitere Aktorik umfassen und zwar insbesondere einen pneumatischen Aktor, wie beispielsweise einen Druckluftaktor, einen mechanischen Aktor, wie beispielsweise einen Schlagwerkaktor, welcher nach Art eines Bohrhammers, und/oder elektromechanischen Aktor, wie beispielsweise nach Art einer Railgun.

Es wird weiter vorgeschlagen, dass das Projektil in einer Ausgangslage zumindest mittelbar an der Sonotrode anliegt. Es kann vorteilhaft eine kompakte Bauart erzielt werden und insbesondere gleichzeitig eine Effizienz einer Energieübertragung verbessert werden. Unter einer "Ausgangslage" soll insbesondere eine Lage verstanden werden, aus welcher das Projektil zu einer Bewegung heraus anregbar ist. Beispielsweise können zwischen Sonotrode und dem Projektil die Verbindungseinheit und/oder das Ultraschallhorn angeordnet sein. Besonders bevorzugt liegt jedoch das Projektil in der Ausgangslage unmittelbar an der Sonotrode an.

Es wird ferner vorgeschlagen, dass die Projektileinheit zumindest ein Stellelement umfasst, welches dazu ausgebildet ist, das Projektil in eine Ausgangslage rückzuführen. Es kann vorteilhaft eine Bewegung verbessert werden. Ferner kann vorteilhaft sichergestellt werden, dass das Projektil in der Ausgangslage wieder angeregt werden kann. Insbesondere presst das Stellelement das Projektil in der Ausgangslage zumindest mittelbar an die Sonotrode an. Das Stellelement ist insbesondere als ein elastisches Element ausgebildet. Das Stellelement beaufschlagt insbesondere eine proximale Seite des Stellelements. Unter einem "elastischen Element" soll insbesondere ein Objekt verstanden werden, dessen Erstreckung entlang zumindest einer Richtung durch eine Verformungskraft entlang dieser Richtung verformbar ist, die sich zumindest im Wesentlichen linear oder linear zu der Erstreckung verhält. Eine Funktion zeigt dabei insbesondere ein zumindest im Wesentlichen lineares Verhalten, wenn jeder Punkt der Funktion zumindest im Wesentlichen zumindest einen Wert, zumindest einer bestimmten linearen Funktion entspricht. Das elastische Element weist insbesondere in der zumindest einen Richtung ein Elastizitätsmodul von höchstens 500 GPa, vorzugsweise höchstens 290 GPa, besonders bevorzugt von höchstens 13 GPa und ganz besonders bevorzugt von höchstens 1 GPa und/oder insbesondere von wenigstens 0,01, vorzugsweise wenigstens 1 GPa, bevorzugt wenigstens 3 GPa und besonders bevorzugt von wenigstens 90 GPa auf. Ferner kann das Stellelement als ein "magnetisches Element" ausgebildet sein. In der Ausgangslage des Projektils weist das Stellelement eine größte Haupterstreckung auf oder ist am weitesten von dem Projektil entfernt. Unter einem "magnetischen Element" soll insbesondere ein Element verstanden werden, welches zumindest teilweise, vorzugsweise zumindest zu einem Großteil und besonders bevorzugt vollständig aus einem magnetisierbaren und/oder paramagnetischen Material besteht und/oder als ein Permanentmagnet oder ein Elektromagnet ausgebildet ist. Um eine magnetische Wechselwirkung mit dem Projektil zu erzielen ist dieses vorzugsweise als ein magnetisches Element ausgebildet. Die Projektileinheit weist insbesondere ein Gegenlager auf, welches zur Abstützung des Stellelements bei einem Einwirken des Stellelements auf das Projektil abstützt. Es ist denkbar, dass das Stellelement und das Projektil miteinander einstückig verbunden sind.

Es wird vorgeschlagen, dass das Stellelement in dem Führungskanal zumindest teilweise, vorzugsweise zumindest zu einem Großteil und besonders bevorzugt vollständig, angeordnet ist. Es kann vorteilhaft eine besonders kompakte Bauform erzielt werden. Ferner ist insbesondere das Gegegenlager an und/oder in dem Führungskanal angeordnet. Ferner ist denkbar, dass das Gegenlager den Fluidkanal zumindest teilweise begrenzt. Alternativ ist denkbar, dass das Stellelement außerhalb des Führungskanals angeordnet ist. Dazu kann das Stellelement beispielsweise den Führungskanal und/oder das Ultraschallhorn in Umfangrichtung umgebend angeordnet sein und zwar insbesondere bei einer Ausgestaltung des Stellelements als ein magnetisches Element.

Das Stellelement ist vorzugsweise als ein elastisches Element ausgebildet, wie beispielsweise ein Bolzen, Zylinder, Schlauch, Ring oder dergleichen. Vorzugsweise umfasst das elastische Element ein elastisches Material, insbesondere Elastomere, wie beispielsweise Gummi oder Silikon. Gemäß einer bevorzugten Ausgestaltung der Erfindung wird vorgeschlagen, dass das Stellelement als eine Feder ausgebildet ist. Es kann vorteilhaft ein Bauraum verringert werden. Ferner bleibt ein Fluidstrom annähernd ungehindert, da dieser derart das elastische Element umströmen kann. Vorzugsweise ist die Feder als eine Druckfeder und/oder Schraubfeder, welche vorzugsweise konisch ausgebildet sein könnte, ausgebildet.

Ferner wird vorgeschlagen, dass die Projektileinheit zumindest ein Fixierelement umfasst, welches dazu ausgebildet ist, in wenigstens einem weiteren Betriebszustand das Projektil in einer Ausgangslage zu fixieren. Es kann vorteilhaft in dem weiteren Betriebszustand eine zusätzlich Anregung der Sonotrode über das Projektil deaktiviert werden, wodurch eine Funktionalität erhöht werden kann. Das Fixierelement könnte als ein elastisches oder mechanisches Element ausgebildet sein. Vorzugsweise ist das Fixierelement als ein magnetisches Element ausgebildet. Das Fixierelement und das Stellelement könnten insbesondere einstückig ausgebildet sein. Ferner ist denkbar, dass das Fixierelement und das Projektil einstückig ausgebildet sind.

Es wird weiter vorgeschlagen, dass das Fixierelement außerhalb des Führungskanals angeordnet ist. Es kann vorteilhaft eine Bedienung verbessert werden, da dies eine Bedienung des Fixierungselements von außen ermöglicht. Vorzugsweise ist das Stellelement den Führungskanal und/oder das Ultraschallhorn in Umfangrichtung umgebend angeordnet.

Es wird vorgeschlagen, dass das Fixierelement als ein Magnet ausgebildet ist. Es kann vorteilhaft eine besonders flexible Betätigung erzielt werden. Bei dem Magneten kann es sich insbesondere um einen Permanentmagneten und/oder einen Elektromagneten handeln. Dazu kann das Stellelement beispielsweise den Führungskanal und/oder das Ultraschallhorn in Umfangrichtung umgebend angeordnet sein und zwar insbesondere bei einer Ausgestaltung des Stellelements als ein magnetisches Element. In einer Fixierstellung des Fixierelements sind insbesondere magnetische Pole des Fixierelements und des Projektils gleichsinnig orientiert, sodass sich diese anziehen.

Es wird vorgeschlagen, dass die Projektileinheit zur Einstellung einer Schlagleistung einer Bewegung des Projektils ausgebildet ist. Es kann vorteilhaft eine Flexibilität verbessert werden. Unter einer "Schlagleistung" soll insbesondere die von der Projektileinheit binnen einer Zeitspanne umgesetzte Energie bezogen auf diese Zeitspanne verstanden werden. Die Schlagleistung ist dabei abhängig von einer Schlagamplitude und/oder der Schlagfrequenz. Insbesondere durch Einstellen einer von dem Stellelement auf das Projektil wirkenden Kraft kann die Schlagleistung insbesondere durch Einstellen der Schlagamplitude und/oder der Schlagfrequenz eingestellt werden. Beispielsweise kann bei einer Ausgestaltung des Stellelements als ein elastisches Element eine von diesem Stellelement auf das Projektil wirkende Vorspannung variiert werden, um die Schlagleistung einzustellen. Ferner ist denkbar, dass eine Schlagamplitude durch Begrenzen des Führungskanals einstellbar ist. Ferner ist denkbar, dass eine Schlagintensität durch Variation der von dem Ultraschallgenerator bereitgestellten Ultraschallwellen einstellbar ist.

Ferner wird ein Lithotripter, insbesondere ein intrakorporaler Lithotripter, mit wenigstens einer Lithotripsievorrichtung vorgeschlagen. Hierdurch kann insbesondere eine Effizienz verbessert werden. Vorteilhaft kann ein Bauraum verringert werden. Weiter vorteilhaft kann eine Leistung zur Zertrümmerung von Konkrementen verbessert werden.

Weiterhin wird ein Lithotripsiesystem mit wenigstens einem Lithotripter vorgeschlagen. Hierdurch kann insbesondere eine Effizienz verbessert werden. Vorteilhaft kann ein Bauraum verringert werden. Weiter vorteilhaft kann eine Leistung zur Zertrümmerung von Konkrementen verbessert werden. Ferner kann das System wenigstens ein Steuergerät und/oder wenigstens eine Fluidpumpe umfassen.

Zudem wird ein Verfahren zum extrakorporalen Testbetrieb einer Lithotripsievorrichtung vorgeschlagen. Hierdurch kann insbesondere eine Effizienz verbessert werden. Vorteilhaft kann ein Bauraum verringert werden. Weiter vorteilhaft kann eine Leistung zur Zertrümmerung von Konkrementen verbessert werden. Unter einem "extrakorporalen Testbetrieb" soll explizit ein Betrieb der Lithotripsievorrichtung außerhalb einer Körperöffnung eines Patienten verstanden werden.

Die erfindungsgemäße Vorrichtung und/oder das erfindungsgemäße Verfahren zum Betrieb einer Vorrichtung sollen/soll hierbei nicht auf die oben beschriebene Anwendung und Ausführungsform beschränkt sein. Insbesondere kann die erfindungsgemäße endoskopische Vorrichtung, das erfindungsgemäße endoskopisches Instrument und/oder das erfindungsgemäße Verfahren zu Betrieb einer endoskopischen Vorrichtung, sowie das erfindungsgemäße Verfahren zur Herstellung einer endoskopischen Vorrichtung zu einer Erfüllung einer hierin beschriebenen Funktionsweise eine von einer hierin genannten Anzahl von einzelnen Elementen, Bauteilen und Einheiten, sowie Verfahrensschritten abweichende Anzahl aufweisen. Zudem sollen bei den in dieser Offenbarung angegebenen Wertebereichen auch innerhalb der genannten Grenzen liegende Werte als offenbart und als beliebig einsetzbar gelten.

Es wird insbesondere darauf hingewiesen, dass alle in Bezug auf die Vorrichtung beschriebenen Merkmale und Eigenschaften aber auch Verfahrensweisen sinngemäß auf das erfindungsgemäße Verfahren übertragbar und im Sinne der Erfindung einsetzbar und als mitoffenbart gelten. Gleiches gilt auch in umgekehrter Richtung. Das bedeutet, dass auch in Bezug auf das Verfahren genannte, bauliche, also vorrichtungsgemäße Merkmale im Rahmen der Vorrichtungsansprüche berücksichtigt, beansprucht und ebenfalls zur Offenbarung gezählt werden können.

### Zeichnungen

Weitere Vorteile ergeben sich aus der folgenden Zeichnungsbeschreibung. In den Zeichnungen sind Ausführungsbeispiele der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: eine schematische Darstellung eines Lithotripsiesystems mit einer Lithotripsievorrichtung in einer perspektivischen Ansicht,
- Fig. 2: eine schematische Darstellung eines Teils der Lithotripsievorrichtung in einer Schnittansicht,
- Fig. 3: einen schematischen Ablaufplan eines beispielhaften Verfahrens zu einem extrakorporalen Testbetrieb der Lithotripsievorrichtung,
- Fig. 4a: eine schematische Darstellung einer alternativen Ausgestaltung einer Lithotripsievorrichtung in einer Fixierstellung in einer Schnittansicht,
- Fig. 4b: eine schematische Darstellung der einer Lithotripsievorrichtung aus Fig. 4a in einer Freigabestellung in einer Schnittansicht,
- Fig. 5: eine schematische Darstellung einer alternativen Ausgestaltung einer Lithotripsievorrichtung in einer Fixierstellung in einer Schnittansicht,,
- Fig. 6: eine schematische Darstellung einer alternativen Ausgestaltung einer Lithotripsievorrichtung in einer Fixierstellung in einer Schnittansicht,
- Fig. 7: eine schematische Darstellung einer alternativen Ausgestaltung einer Lithotripsievorrichtung in einer Fixierstellung in einer Schnittansicht,
- Fig. 8: eine schematische Darstellung einer alternativen Ausgestaltung einer Lithotripsievorrichtung in einer Fixierstellung in einer Schnittansicht,
- Fig. 9: eine schematische Darstellung einer alternativen Ausgestaltung einer Lithotripsievorrichtung in einer Fixierstellung in einer Schnittansicht,und
- Fig. 10: eine schematische Darstellung einer alternativen Ausgestaltung einer Lithotripsievorrichtung in einer Schnittansicht.

### Beschreibung der Ausführungsbeispiele

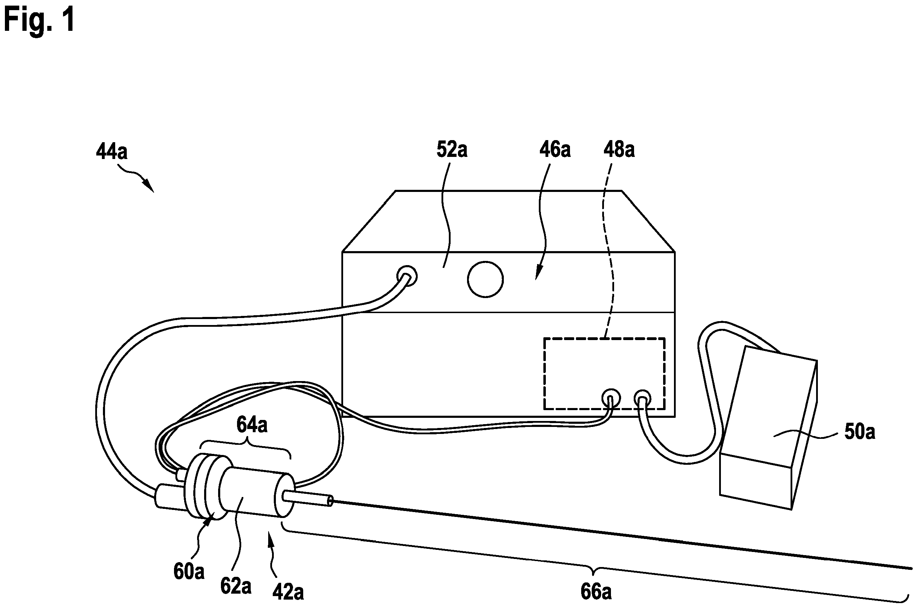

Fig. 1 zeigt eine schematische Darstellung eines Lithotripsiesystems 44a in einer perspektivischen Ansicht. Das Lithotripsiesystem 44a weist wenigstens einen Lithotripter 42a mit einer Lithotripsievorrichtung auf. Der Lithotripter 42a ist im vorliegenden Fall als ein intrakorporaler Lithotripter ausgebildet. Alternativ könnte der Lithotripter 42a auch als ein extrakorporaler Lithotripter 42a ausgebildet sein. Die Lithotripsievorrichtung bildet im vorliegenden Fall den Lithotripter 42a vollständig aus. Alternativ könnte die Lithotripsievorrichtung auch nur ein Teil des Lithotripters 42a ausbilden.

Das Lithotripsiesystem 44a weist wenigstens ein Steuergerät 46a auf. Das Steuergerät 46a umfasst zumindest eine Steuereinheit 48a auf. Die Steuereinheit 48a ist zu einer Ansteuerung der Lithotripsievorrichtung ausgebildet. Die Steuereinheit 48a umfasst wenigstens eine Steuerelektronik. Die Steuerelektronik weist zumindest einen Prozessor auf. Ferner weist die Steuerelektronik wenigstens einen Speicher auf. Auf dem Speicher ist ein Betriebsprogramm hinterlegt. Das Betriebsprogramm ist von dem Prozessor ausführbar. Der Lithotripsievorrichtung ist mit dem Steuergerät 46a verbunden und zwar insbesondere mit der Steuerelektronik.

Das Lithotripsiesystem 44a weist wenigstens ein Betätigungsmittel 50a auf. Das Betätigungsmittel 50a ist zu einer Aktivierung und/oder Deaktivierung des Lithotripters 42a ausgebildet. Das Betätigungsmittel 50a ist mit dem Steuergerät 46a und zwar insbesondere mit der Steuerelektronik des Steuergeräts 46a verbunden. Im vorliegenden Fall ist das Betätigungsmittel 50a als ein Fußschalter ausgebildet. Alternativ könnte das Betätigungsmittel auch als ein anderer elektrischer und/oder elektronischer Schalter ausgebildet sein, wie beispielsweise als ein Druckknopf, einen Kippschalter oder dergleichen, welcher insbesondere auch an dem Lithotripter 42a selbst angeordnet sein könnte. Auch könnte eine Bedienung des Lithotripters 42a über ein externes Gerät, insbesondere ein Handgerät, wie beispielsweise ein Tablet, ein Smartphone, eine Smartwatch oder dergleichen erfolgen.

Das Lithotripsiesystem 44a weist ferner eine Fluidpumpe 52a auf. Die Fluidpumpe 52a ist zur Förderung eines Fluids ausgebildet. Die Fluidpumpe 52a dient zum Absaugen und/oder Freispulen bei einem Lithotripsievorgang. Beispielsweise können mit dem Fluid Teile eines bei einem Lithotripsievorgang zerstörten Konkrements anfallenden Bruchteile entfernt werden. Dazu ist die Fluidpumpe 52a fluidtechnisch mit der Lithotripsievorrichtung verbunden.

Die Lithotripsievorrichtung weist einen proximalen Abschnitt 64a auf. Der proximale Abschnitt 64a ist bei einer Bedienung einem Bediener der Lithotripsievorrichtung zugewandt. Der proximale Abschnitt 64a ist bei einer Bedienung einem Patienten abgewandt. Der proximale Abschnitt 64a liegt bei einer Behandlung eines Patienten außerhalb des Patienten. Ferner weist die Lithotripsievorrichtung einen distalen Abschnitt 66a auf. Der distale Abschnitt 66a ist bei einer Bedienung einem Bediener der Lithotripsievorrichtung abgewandt. Der distale Abschnitt 66a ist bei einer Bedienung einem Patienten zugewandt. Der distale Abschnitt 66a liegt bei einer Behandlung eines Patienten zumindest teilweise innerhalb des Patienten.

Die Lithotripsievorrichtung weist ein Gehäuse 60a auf. Das Gehäuse 60a dient zur Anordnung weiterer Komponenten der Lithotripsievorrichtung. Das Gehäuse 60a bildet zumindest teilweise den proximalen Abschnitt 64 der Lithotripsievorrichtung aus. Eine Haupterstreckung des Gehäuses 60a entspricht zumindest im Wesentlichen einer Haupterstreckung des proximalen Abschnitts 64a.

Ferner weist die Lithotripsievorrichtung eine Handhabe 62a auf. Die Handhabe 62a bildet zumindest teilweise den proximalen Abschnitt 64a aus. Die Handhabe 62a ist zu einer manuellen Bedienung der Lithotripsievorrichtung ausgebildet. Die Handhabe 62a ist von dem Gehäuse 60a ausgebildet. Alternativ könnte insbesondere bei einer robotischen Ansteuerung der Lithotripsievorrichtung auch auf eine Handhabe 62a verzichtet werden.

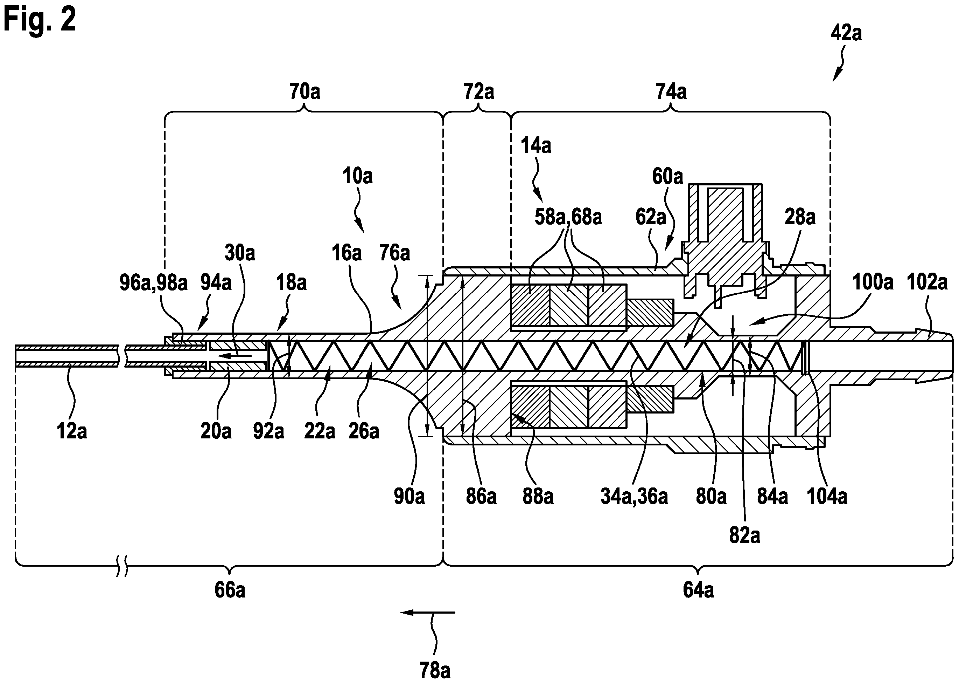

Fig. 2 zeigt eine schematische Darstellung eines Teils der Lithotripsievorrichtung in einer Schnittansicht. Die Lithotripsievorrichtung umfasst wenigstens eine Ultraschalleinheit 10a. Die Ultraschalleinheit 10a dient zu einer auf Ultraschall basierenden Zertrümmerung von Konkrementen, wie beispielsweise Gallenblase, Nieren, Harnleiter, Blase oder dergleichen. Die Ultraschalleinheit 10a ist zumindest teilweise in dem Gehäuse 60a angeordnet. Die Ultraschalleinheit 10a bildet zumindest teilweise den proximalen Abschnitt 64a der Lithotripsievorrichtung aus. Die Ultraschalleinheit 10a ist zumindest teilweise außerhalb des Gehäuses 60a angeordnet. Die Ultraschalleinheit 10a bildet zumindest teilweise den distalen Abschnitt 66a der Lithotripsievorrichtung aus.

Die Ultraschalleinheit 10a weist wenigstens einen Ultraschallgenerator 14a auf. Der Ultraschallgenerator 14a ist dazu ausgebildet, Ultraschallwellen bereitzustellen. Zu einer Ansteuerung ist der Ultraschallgenerator 14a mit der Steuerelektronik verbunden. Der Ultraschallgenerator 14a ist zumindest teilweise in dem Gehäuse 60a angeordnet. Im vorliegenden Fall ist der Ultraschallgenerator 14a vollständig in dem Gehäuse 60a angeordnet. Der Ultraschallgenerator 14a bildet zumindest teilweise den proximalen Abschnitt 64a der Lithotripsievorrichtung aus.

Der Ultraschallgenerator 14a weist wenigstens einen Ultraschallaktor 58a. Im vorliegenden Fall weist der Ultraschallgenerator 14a mehrere Ultraschallaktoren 58a auf. Der Ultraschallgenerator 14a weist drei Ultraschallaktoren 58a auf. Die Ultraschallaktoren 58a sind aneinander anliegend gestapelt angeordnet. Der Übersichtlichkeit halber ist in den Zeichnungen nur ein Ultraschallaktor 58a mit einem Bezugszeichen versehen. Die Ultraschallaktoren 58a sind zumindest im Wesentlichen identisch zueinander ausgebildet. Im Folgenden ist nur ein Ultraschallaktor 58a näher beschrieben. Diese Beschreibung ist auch auf die weiteren Ultraschallaktoren 58a übertragbar. Der Ultraschallaktor 58a weist die Form eines Hohlzylinders auf. Im vorliegenden Fall ist der Ultraschallaktor 58a als ein Piezoaktor 68a ausgebildet. Im vorliegenden Fall sind der Übersichtlichkeit halber etwaige Anschluss- und/oder Kontaktierungsmittel und/oder Isolationen der Ultraschallaktoren 58a nicht in der Fig. 2 dargestellt.

Zudem weist die Ultraschalleinheit 10a wenigstens ein Ultraschallhorn 16a auf. Das Ultraschallhorn 16a ist zur Übertragung von Ultraschallwellen ausgebildet. Das Ultraschallhorn 16a ist zur Fokussierung von Ultraschallwellen ausgebildet. Das Ultraschallhorn 16a ist zumindest teilweise in dem Gehäuse 60a angeordnet. Das Ultraschallhorn 16a bildet zumindest teilweise den proximalen Abschnitt 64a der Lithotripsievorrichtung aus. Das Ultraschallhorn 16a erstreckt sich über wenigstens einen Großteil des distalen Abschnitts 66a der Lithotripsievorrichtung. Das Ultraschallhorn 16a ist zumindest teilweise außerhalb des Gehäuse 60a angeordnet. Das Ultraschallhorn 16a bildet zumindest teilweise den distalen Abschnitt 66a der Lithotripsievorrichtung aus. Das Ultraschallhorn 16a weist die Form eines Rotationskörpers auf. Im vorliegenden Fall weist das Ultraschallhorn 16a eine hohlzylinderartige Form auf. Das Ultraschallhorn 16a besteht zumindest teilweise aus Metall, insbesondere Stahl oder Titan.

Das Ultraschallhorn 16a weist eine Ausnehmung 80a auf. Die Ausnehmung 80a erstreckt sich von einem proximalen Ende des Ultraschallhorns 16a bis zu einem distalen Ende des Ultraschallhorns 16a. Die Ausnehmung 80a weist einen Innendurchmesser 82a auf. Die Ausnehmung 80a verläuft axial entlang einer Rotationsachse des Ultraschallhorns 16a.

Das Ultraschallhorn 16a weist einen proximalen Endabschnitt 74a auf. Der proximalen Endabschnitt 74a ist innerhalb des Gehäuses 60a angeordnet. Der proximale Endabschnitt 74a ist innerhalb des proximalen Abschnitts 64a der Lithotripsievorrichtung angeordnet. Der proximale Endabschnitt 74a bildet zumindest teilweise den proximalen Abschnitt 64a der Lithotripsievorrichtung aus. Der proximalen Endabschnitt 74a weist einen Außendurchmesser 84a auf. Ein Innendurchmesser 82a der Ausnehmung 80a des Ultraschallhorns 16a ist im Wesentlichen größer als eine Differenz des Außendurchmessers 84a und des Innendurchmessers 82a. Im vorliegende Fall ist der Innendurchmesser 82a der Ausnehmung 80a zumindest um einen Faktor zwei größer als eine Differenz des Außendurchmessers 84a und des Innendurchmessers 82a. Es kann vorteilhaft eine Schwingungsentkopplung von weiteren Anschlüssen, insbesondere dem Fluidanschluss, relativ zum Ultraschallhorn erzielt werden. Der proximale Endabschnitt 74a ist zumindest teilweise, insbesondere durch die Dünnwandige Ausgestaltung, elastisch ausgebildet.

Das Ultraschallhorn 16a weist einen Mittelabschnitt 72a auf. Der Mittelabschnitt 72a erstreckt sich zwischen dem proximalen Endabschnitt 74a und dem distalen Endabschnitt 70a. Der Mittelabschnitt 72a ist innerhalb des proximalen Abschnitts 64a der Lithotripsievorrichtung angeordnet. Der Mittelabschnitt 72a bildet zumindest teilweise den proximalen Abschnitt 64a der Lithotripsievorrichtung aus. Der Mittelabschnitt 72a weist einen weiteren Außendurchmesser 86a auf. Der Innendurchmesser 82a der Ausnehmung 80a ist im Wesentlichen kleiner als eine Differenz des weiteren Außendurchmessers 86a und des Innendurchmessers 82a der Ausnehmung 80a. Im vorliegenden Fall ist der Innendurchmesser 82a der Ausnehmung 80a zumindest um einen Faktor zwei kleiner als eine Differenz des weiteren Außendurchmessers 86a und des Innendurchmessers 82a der Ausnehmung 80a.

Das Ultraschallhorn 16a weist eine Stufe 88a auf. Die Stufe 88a bildet einen Übergang zwischen dem Mittelabschnitt 72a und dem proximalen Endabschnitt 74a aus. Die Stufe 88a weist eine Höhe auf, welche der Differenz zwischen dem weiteren Außendurchmesser 86a des Mittelabschnitts 72a und dem Außendurchmesser 84a des proximalen Endabschnitts 74a entspricht.

Der Ultraschallgenerator 14a ist das Ultraschallhorn 16a zumindest teilweise umgebend angeordnet. Der Ultraschallgenerator 14a ist im Bereich des proximalen Endabschnitts 74ades Ultraschallhorns 16a angeordnet. Der Ultraschallgenerator 14a liegt an der Stufe 88a an. Der Ultraschallgenerator 14a schließt bündig mit dem weiteren Außendurchmesser 86a des Mittelabschnitts 72a ab.

Das Ultraschallhorn 16a weist einen distalen Endabschnitt 70a auf. Der distale Endabschnitt 70a ist außerhalb des Gehäuses 60a angeordnet. Der distale Endabschnitt 70a ist außerhalb des proximalen Abschnitts 64a der Lithotripsievorrichtung angeordnet. Der distale Endabschnitt 70a ist innerhalb des distalen Abschnitts 66a der Lithotripsievorrichtung angeordnet. Der distale Endabschnitt 70a bildet zumindest teilweise den distalen Abschnitt 66a der Lithotripsievorrichtung aus. Der distale Endabschnitt 70a weist einen Außendurchmesser 90a auf. Der Außendurchmesser 90a entspricht im vorliegenden Fall dem Außendurchmesser 86a des Mittelabschnitts 72a. Ferner weist der distale Endabschnitt 70a einen weiten Außendurchmesser 92a auf. Der weitere Außendurchmesser 92a entspricht im vorliegenden Fall dem Außendurchmesser 84a des proximalen Endabschnitts 74a.

Das Ultraschallhorn 16a weist eine Verjüngung 76a auf. Die Verjüngung 76a liegt im Bereich des distalen Endabschnitts 70a. Die Verjüngung 76a ist frei von Stufen. Die Verjüngung 76a weist einen stetigen Verlauf auf. Der Verlauf der Verjüngung 76a ist konkav. Der Verlauf der Verjüngung 76a ist exponentiell. Zur Ausbildung der Verjüngung 76a verjüngt sich der distale Endabschnitt 70a in distaler Richtung 78a. Die Verjüngung 76a verjüngt sich um einen Betrag, welcher der Differenz zwischen dem Außendurchmesser 90a und dem weiteren Außendurchmesser 92a des distalen Endabschnitts 70a entspricht. Alternativ oder zusätzlich könnte die Verjüngung auch stufenförmig oder katenoidalförmig sein.

Die Ultraschalleinheit 10a weist wenigstens eine Sonotrode 12a auf. Die Sonotrode 12a ist zumindest teilweise außerhalb des Gehäuse 60a angeordnet. Im vorliegenden Fall ist die Sonotrode 12a vollständig außerhalb des Gehäuses 60a angeordnet. Die Sonotrode 12a bildet zumindest zu einem Großteil den distalen Abschnitt 66a der Lithotripsievorrichtung aus. Die Sonotrode 12a weist die Form eines Rohrs auf. Die Sonotrode 12a ist starr ausgebildet. Die Sonotrode 12a besteht zumindest teilweise aus Metall, insbesondere Stahl.

Die Sonotrode 12a ist zumindest teilweise in dem Ultraschallhorn 16a angeordnet und zwar insbesondere in dem distalen Endabschnitt 70a. Das Ultraschallhorn 16a ist zu einer zumindest mittelbaren Übertragung der Ultraschallwellen von dem Ultraschallgenerator 14a auf die Sonotrode 12a ausgebildet. Die Sonotrode 12 ist mit dem Ultraschallhorn 16a verbunden. Zur Verbindung weist die Ultraschalleinheit 10a wenigstens eine Verbindungseinheit 94a auf. Die Verbindungseinheit 94a ist zu einer kraft- und/oder formschlüssigen Verbindung der Sonotrode 12a mit dem Ultraschallhorn 16a ausgebildet. Die Verbindungseinheit 94a weist zumindest ein Verbindungselement 96a auf. Das Verbindungselement 96a weist die Form einer Hülse auf, welche einen freien proximalseitigen Endabschnitt der Sonotrode 12a umschließt. Das Verbindungselement 96a ist im vorliegenden Fall als ein Gewinde 98a ausgebildet.

Die Lithotripsievorrichtung umfasst wenigstens eine Projektileinheit 18a. Die Projektileinheit 18a umfasst wenigstens ein Projektil 20a. Das Projektil 20a ist linear beweglich gelagert. In wenigstens einem Betriebszustand ist das Projektil 20a dazu ausgebildet, die Sonotrode 12a zumindest mittelbar zu beaufschlagen. Im vorliegenden Fall beaufschlagt das Projektil 20a die Sonotrode 12a sogar unmittelbar. Alternativ könnte die Sonotrode 12a auch die Verbindungseinheit 94a, welche die Sonotrode 12a mit dem Ultraschallhorn 16a verbindet, und/oder das Ultraschallhorn 16a beaufschlagen, welche/welches dann wiederum die Beaufschlagung an die Sonotrode 12a übertragen/überträgt. Das Projektil 20a ist im Bereich des Ultraschallhorns 16a beweglich gelagert angeordnet. Das Projektil 20a ist in einer Ausgangsstellung im distalen Endabschnitt 70a des Ultraschallhorns 16a angeordnet.

Die Projektileinheit 18a weist wenigstens einen Führungskanal 22a auf. Der Führungskanal 22a ist zumindest zur beweglichen Lagerung des Projektils 20a ausgebildet. Der Führungskanal 22a ist zumindest teilweise von der Ultraschalleinheit 10a ausgebildet. Im vorliegenden Fall ist der Führungskanal 22a zumindest teilweise von dem Ultraschallhorn 16a ausgebildet. Das Ultraschallhorn 16a der Ultraschalleinheit 10a weist wenigstens einen Führungsabschnitt 26a auf. Der Führungsabschnitt 26a bildet zumindest teilweise den Führungskanal 22a aus. Im vorliegenden Fall bildet der Führungsabschnitt 26a den Führungskanal 22a vollständig aus. Der Führungsabschnitt 26a ist von der Ausnehmung 80a des Ultraschallhorns 16a ausgebildet. Alternativ oder zusätzlich könnten auch weitere Komponenten der Ultraschalleinheit 10a den Führungskanal 22a ausbilden.

Die Lithotripsievorrichtung umfasst wenigstens eine Fluideinheit 100a. Die Fluideinheit 100a ist dazu ausgebildet, Fluid zum Spülen und/oder Absaugen bereit zu stellen. Die Fluideinheit 100a ist mit der Fluidpumpe 52a fluidtechnisch verbunden. Dazu weist die Fluideinheit 100a einen Fluidanschluss 102a auf. Der Fluidanschluss 102a ist am Ende des proximalen Abschnitts 64a der Lithotripsievorrichtung angeordnet. Die Fluideinheit 100a weist wenigstens einen Fluidkanal 28a auf. Der Fluidkanal 28a erstreckt sich von dem proximalen Abschnitt 64a bis zum distalen Abschnitt 66a der Lithotripsievorrichtung. Der Fluidkanal 28a ist zumindest teilweise von der Sonotrode 12a ausgebildet. Ferner ist der Fluidkanal 28a zumindest teilweise von der Ausnehmung 80a des Ultraschallhorns 16a ausgebildet. Demnach bildet der Führungskanal 22a zumindest teilweise den Fluidkanal 28a aus. Im vorliegenden Fall ist der Führungskanal 22a im Bereich des Ultraschallhorns 16a sogar mit dem Fluidkanal 28a identisch.

Da das Projektil 20a in dem Bereich des Ultraschallhorns 16a im Fluidkanal 28a angeordnet ist, weist das Projektil 20a wenigstens einen Fluiddurchlass 30a auf, um einen Fluidfluss durch den Fluidkanal 28a zu ermöglichen.

In der Ausgangslage liegt das Projektil 20a im vorliegenden Fall unmittelbar an der Sonotrode 12a an. Das Projektil 20a wird in einem Betriebszustand zumindest mittelbar von dem Ultraschallgenerator 14a zu einer Bewegung angeregt. Das Projektil 20a ist über das Ultraschallhorn 16a erregbar. Dabei übertragen sich Ultraschallwellen von dem Ultraschallgenerator 14a auf die Sonotrode 12a und von der Sonotrode 12a auf das Projektil 20a.

Die Projektileinheit 18a weist zumindest ein Stellelement 34a auf. Das Stellelement 34a ist dazu ausgebildet, bei einer Anregung das Projektil 20a in die Ausgangslage rückzuführen. Das Stellelement 34a ist zumindest teilweise in dem Führungskanal 22a angeordnet. Das Stellelement 34a ist als ein elastisches Element ausgebildet. Im vorliegenden Fall ist das Stellelement 34a als eine Feder 36, insbesondere Druckfeder, ausgebildet. Alternativ kann das Stellelement 34a auch als ein anderes elastisches Element ausgebildet sein, wie beispielsweise ein Gummiring oder dergleichen.

Die Projektileinheit 18a ist zur Einstellung einer Schlagleistung einer Bewegung des Projektils 20a ausgebildet. Im vorliegenden Fall kann ist eine Vorspannung des Stellelements 34a einstellbar, wodurch eine Rückstellkraft des Stellelements 34a und somit auch die Schlagleistung einstellbar ist.

Die Projektileinheit 18a weist zumindest ein Gegenlager 104a auf. Das Gegenlager 104a ist dazu ausgebildet, dass das Stellelement 34a sich auf diesem gegen eine bei einer der Rückstellung des Projektils 20a wirkende Kraft abstützen kann. Das Gegenlager 104a ist in dem Führungskanal 22a angeordnet.

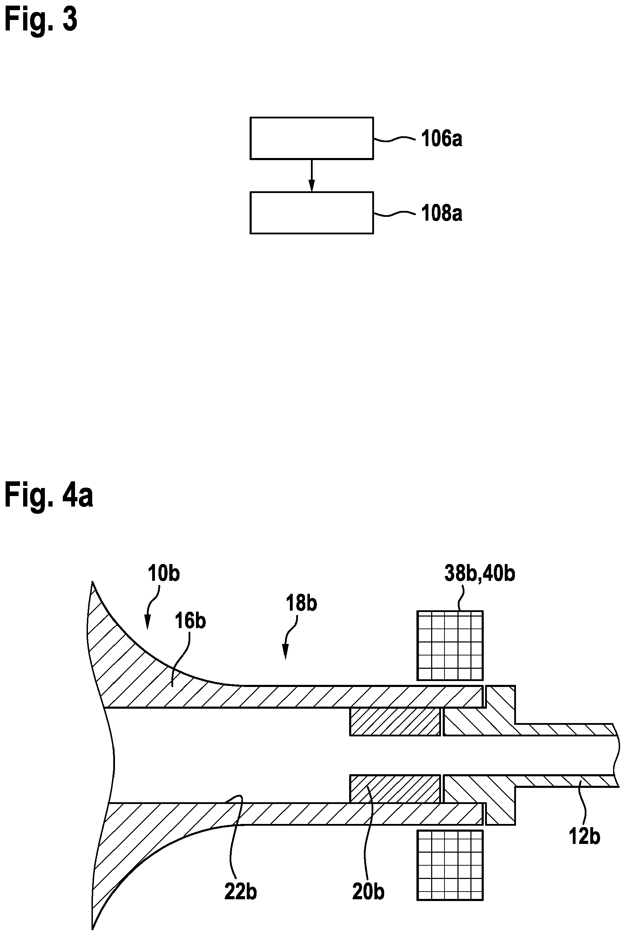

Fig. 3 zeigt einen schematischen Ablaufplan eines beispielhaften Verfahrens zum extrakorporalen Testbetrieb der Lithotripsievorrichtung. Das Verfahren zum extrakorporalen Testbetrieb der Lithotripsievorrichtung ist Teil des Betriebsprogramms.

Das Verfahren umfasst zumindest einen Verfahrensschritt 106a. In dem Verfahrensschritt 106a wird das Projektil 20a von dem Stellelement 34a in die Ausgangslage gestellt. Das Projektil 20a liegt in der Ausgangslage an der Sonotrode 12a an.

Das Verfahren umfasst wenigstens einen weiteren Verfahrensschritt 108a. In dem weiteren Verfahrensschritt 108a wird der Ultraschallgenerator 14a aktiviert. Der Ultraschallgenerator 14a stellt Ultraschallwellen bereit. Die Ultraschallwellen werden von dem Ultraschallhorn 16a von dem Ultraschallgenerator 14a auf die Sonotrode 12a übertragen und fokussiert. Die Sonotrode 12a überträgt die Ultraschallwellen wiederum auf das Projektil 20a. Das Projektil 20a wird durch die Ultraschallwellen zu einer Bewegung angeregt. Bei der Bewegung kann es sich um eine, insbesondere erzwungene, Schwingungsbewegung handeln. Die Bewegung weist dabei eine Stoßfrequenz auf, welche wesentlich kleiner ist die anregende Ultraschallfrequenz. Im vorliegenden Fall ist die Stoßfrequenz der Bewegung zumindest um einen Faktor 100 geringer als die Ultraschallfrequenz. Führt das Projektil 20a eine Bewegung aus, wird dieses durch das Stellelement 34a in die Ausgangslage rückgestellt. Das Projektil 20a trifft auf die Sonotrode 12a und überträgt einen Stoß auf die Sonotrode 12a.

Die Verfahrensschritte 106a und 108a werden entsprechend einer Anzahl von benötigten Stößen durch das Projektil 20a wiederholt. Beispielsweise kann eine Anzahl von benötigten Stößen anhand einer Größe eines zu zertrümmernden Konkrements und somit mittels des Testbetriebs eine Dauer eines Eingriffs abgeschätzt werden.

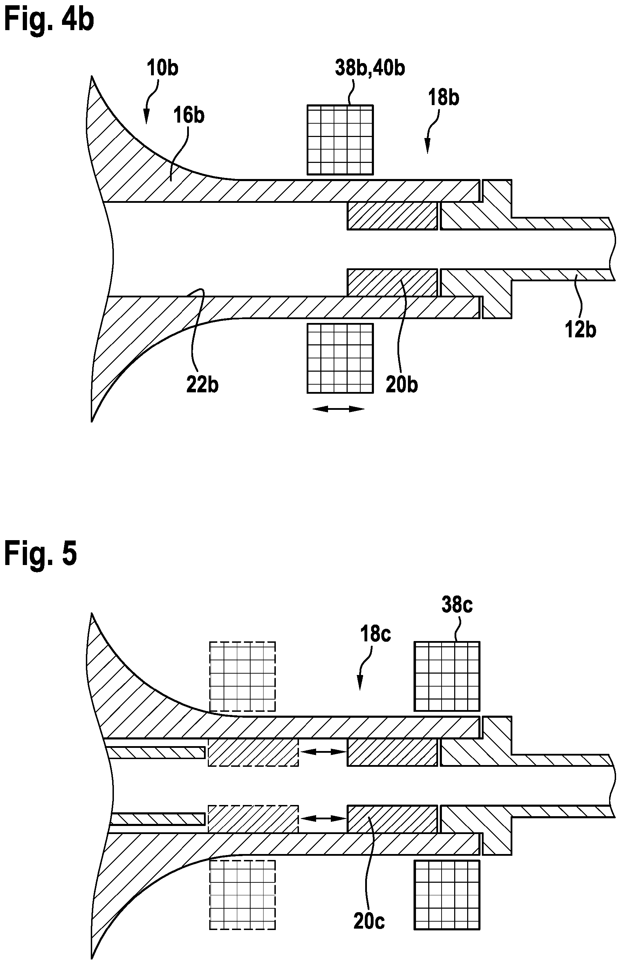

In den Figuren 4 bis 10 sind weitere Ausführungsbeispiele der Erfindung gezeigt. Die nachfolgenden Beschreibungen und die Zeichnungen beschränken sich im Wesentlichen auf die Unterschiede zwischen den Ausführungsbeispielen, wobei bezüglich gleich bezeichneter Bauteile, insbesondere in Bezug auf Bauteile mit gleichen Bezugszeichen, grundsätzlich auch auf die Zeichnungen und/oder die Beschreibung der anderen Ausführungsbeispiele, insbesondere der Figuren 1 bis 3 verwiesen wird. Zur Unterscheidung der Ausführungsbeispiele ist der Buchstabe a den Bezugszeichen des Ausführungsbeispiels in den Figuren 1 bis 3 nachgestellt. In den Ausführungsbeispielen der Figuren 4 bis 10 ist der Buchstabe a durch die Buchstaben b bis h ersetzt.

Fig. 4 zeigt eine alternative Ausgestaltung einer Lithotripsievorrichtung. Das vorliegende Ausführungsbeispiel der Lithotripsievorrichtung unterscheidet sich im Wesentlichen von der Vorhergehenden durch eine Ausgestaltung einer Projektileinheit 18b der Lithotripsievorrichtung.

Im vorliegenden Fall weist die die Projektileinheit 18b zumindest ein Fixierelement 38b auf. Das Fixierelement 38b ist dazu ausgebildet, in wenigstens einem weiteren Betriebszustand ein Projektil 20b der Projektileinheit 18b in einer Ausgangslage zu fixieren. Dadurch kann gezielt eine Bewegung des Projektils 20a auch bei eine Aktivierung des Ultraschallgenerators (nicht dargestellt) einer Ultraschalleinheit 10b verhindert werden, wodurch in dem weiteren Betriebszustand eine vorzugsweise ausschließliche Beaufschlagung einer Sonotrode 12b der Ultraschalleinheit 10b mit Ultraschallwellen erfolgt und zwar insbesondere ohne zusätzliche Stöße des Projektils 20a.

Das Fixierelement 38b ist im vorliegenden Fall außerhalb eines Führungskanals 22b der Projektileinheit 18b angeordnet. Das Fixierelement 38b umschließt das Ultraschallhorn 16b in Umfangsrichtung. Das Fixierelement 38b ist axial verschiebbar gelagert entlang einer Haupterstreckung eines Ultraschallhorns 16b der Ultraschalleinheit 10b. Das Fixierelement 38b weist eine Fixierstellung auf. In der Fixierstellung fixiert das Fixierelement das Projektil einer Ausgangslage (vgl. Fig. 4a).

Das Fixierelement 38b ist als ein Magnet 40b ausgebildet. Im vorliegenden Fall handelt es sich bei dem Magneten 40b um einen Permanentmagneten. Ferner ist das Projektil 20b im vorliegenden Fall aus einem magnetischen Material. In der Fixierstellung sind magnetische Pole des Fixierelements 38b und des Projektils 20b gleichsinnig orientiert, sodass sich diese anziehen. Derart kann das Fixierelement 38b mit dem Projektil 20b wechselwirken und dieses in der Ausgangslage fixieren.

Das Fixierelement 38b weist eine Freigabestellung auf (vgl. Fig. 4b). In der Freigabestellung gibt das Fixierelement 38b das Projektil 20b zu einer Bewegung frei. Zur Überführung des Fixierelements 38b von der Fixierstellung in die Freigabestellung ist dieses in proximale Richtung verschiebbar. In der Freigabestellung sind magnetische Pole des Fixierelements 38b und des Projektils 20a gegensinnig orientiert, sodass sich diese abstoßen. Derart kann das Fixierelement 38b mit dem Projektil 20b wechselwirken und dieses aus dessen Ausgangslage freigeben. Ferner wirkt das Fixierelement 38b in der Freigabestellung zusätzlich als ein Stellelement 34b der Projektileinheit 18b, sodass beispielsweise auf ein elastisches Stellelement verzichtet werden kann.

Fig. 5 zeigt eine alternative Ausgestaltung einer Lithotripsievorrichtung. Das vorliegenden Ausführungsbeispiel der Lithotripsievorrichtung unterscheidet sich im Wesentlichen von der Vorhergehenden durch eine Ausgestaltung einer Projektileinheit 18c der Lithotripsievorrichtung. Im vorliegenden Fall ist ein Fixierelement 38c der Projektileinheit 18c als ein Elektromagnet ausgebildet. Das Fixierelement 38c ist schaltbar. Das Fixierelement 38c kann durch Anlegen oder Unterbrechen eines elektrischen Stroms in einen Fixierzustand bzw. einen Freigabezustand überführt werden. In dem Fixierzustand fixiert das Fixierelement 38c ein Projektil 20c der Projektileinheit 18c in der Ausgangslage. Im Freigabezustand gibt das Fixierelement 38c das Projektil 20c zu einer Bewegung frei.

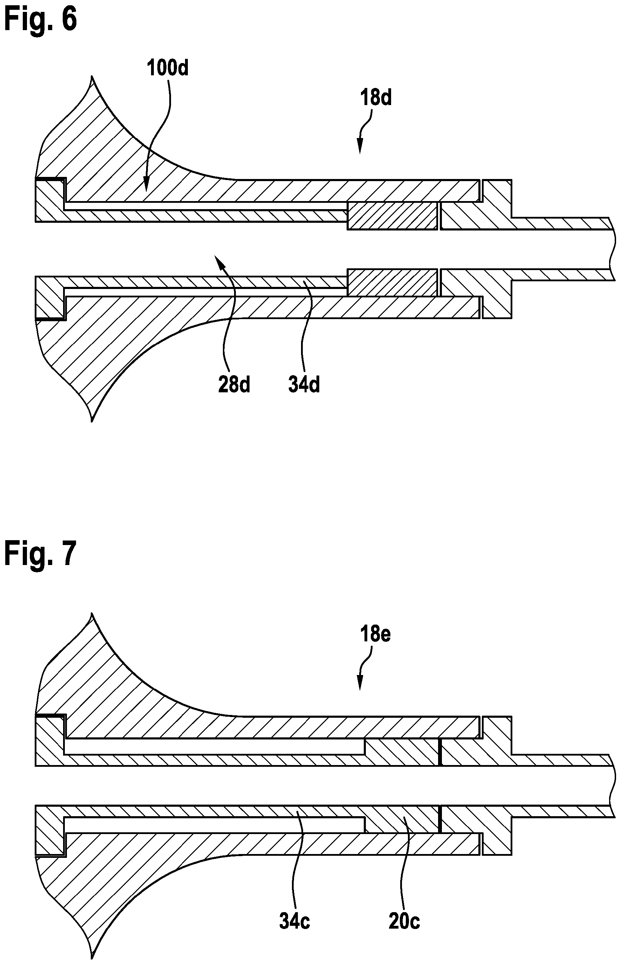

Fig. 6 zeigt eine alternative Ausgestaltung einer Lithotripsievorrichtung. Das vorliegende Ausführungsbeispiel der Lithotripsievorrichtung unterscheidet sich im Wesentlichen von der Vorhergehenden durch eine Ausgestaltung einer Projektileinheit 18d der Lithotripsievorrichtung.

Im vorliegenden Fall weist die Projektileinheit 18d ein Stellelement 34d auf. Das Stellelement 34d ist als ein elastisches Rohr ausgebildet. Das Stellelement 34d bildet im vorliegenden Fall zumindest teilweise einen Fluidkanal 28d einer Fluideinheit 100d der Lithotripsievorrichtung aus.

Fig. 7 zeigt eine alternative Ausgestaltung einer Lithotripsievorrichtung. Das vorliegende Ausführungsbeispiel der Lithotripsievorrichtung unterscheidet sich im Wesentlichen von der Vorhergehenden durch eine Ausgestaltung einer Projektileinheit 18e der Lithotripsievorrichtung.

Im vorliegenden Fall weist eine Projektileinheit 18e der Lithotripsievorrichtung ein Stellelement 34e auf, welches einstückig mit einem Projektil 20e der Projektileinheit 18e verbunden ist.

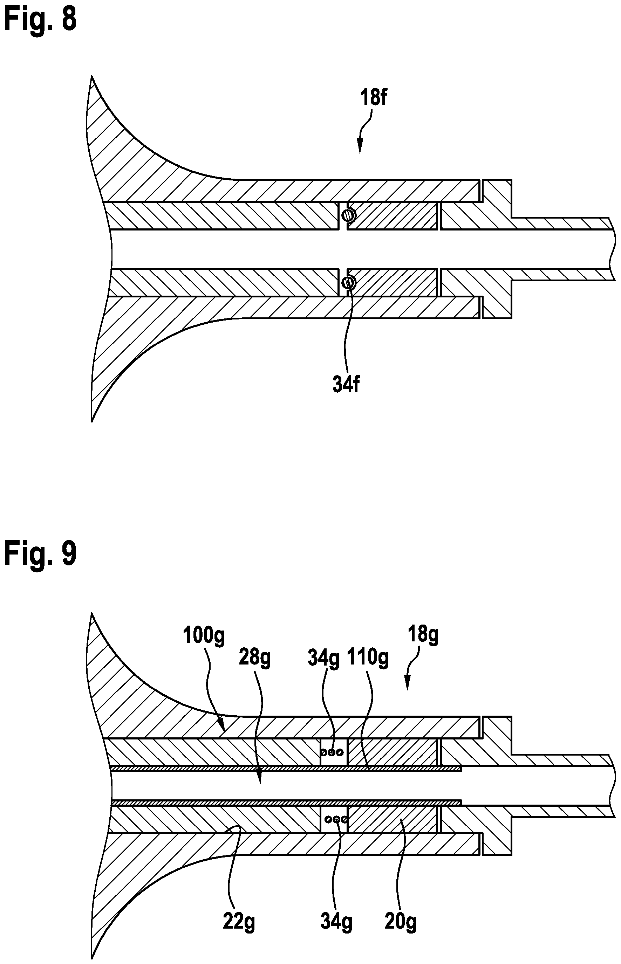

Fig. 8 zeigt eine alternative Ausgestaltung einer Lithotripsievorrichtung. Das vorliegende Ausführungsbeispiel der Lithotripsievorrichtung unterscheidet sich im Wesentlichen von der Vorhergehenden durch eine Ausgestaltung einer Projektileinheit 18f der Lithotripsievorrichtung.

Im vorliegenden Fall ist ein Stellelement 34f der Projektileinheit 18f als ein elastischer O-Ring ausgebildet.

Fig. 9 zeigt eine alternative Ausgestaltung einer Lithotripsievorrichtung. Das vorliegende Ausführungsbeispiel der Lithotripsievorrichtung unterscheidet sich im Wesentlichen von der Vorhergehenden durch eine Ausgestaltung einer Projektileinheit 18g der Lithotripsievorrichtung.

Im vorliegenden Fall weist eine Fluideinheit 100g der Lithotripsievorrichtung ein Abdichtelement 110g auf. Das Abdichtelement 110g ist als ein Rohr ausgebildet. Das Abdichtelement 110g ist in einen Führungskanal 22g der Projektileinheit 18g eingelassen. Das Abdichtelement 110g weist einen Durchmesser auf, welcher kleiner ist als ein Fluiddurchlass eines Projektils 20g der Projektileinheit 18g. Das Abdichtelement 110g ist von dem Projektil 20g umgebend angeordnet. Das Abdichtelement 110g ist von einem Stellelement 34g der Projektileinheit 18g umgebend angeordnet. Im vorliegenden Fall bildet der Führungskanal 34g nicht einen Fluidkanal 28g der Fluideinheit 100g aus. Das Stellelement 34g ist außerhalb des Fluidkanals 28g angeordnet. Das Projektil 20g ist außerhalb des Fluidkanals 28g angeordnet.

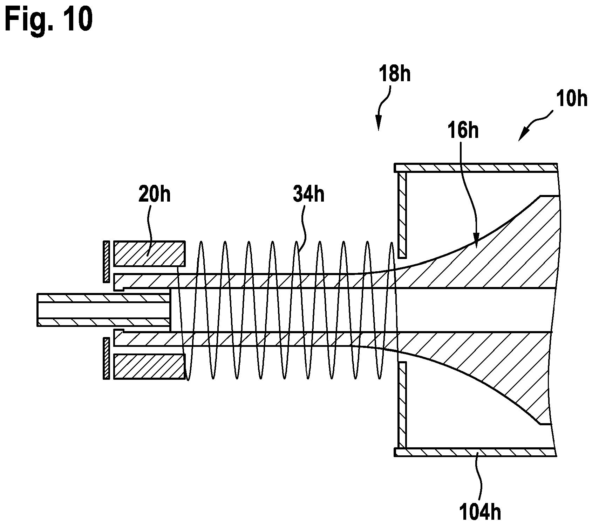

Fig. 10 zeigt eine alternative Ausgestaltung einer Lithotripsievorrichtung. Das vorliegende Ausführungsbeispiel der Lithotripsievorrichtung unterscheidet sich im Wesentlichen von der Vorhergehenden durch eine Ausgestaltung einer Projektileinheit 18h der Lithotripsievorrichtung.

Die Projektileinheit 18h umfasst ein Projektil 20h, welches im Bereich eines Ultraschallhorns 16h einer Ultraschalleinheit der Lithotripsievorrichtung beweglich gelagert angeordnet ist. Im vorliegenden Fall ist das Projektil 20h das Ultraschallhorn umgeben, und zwar insbesondere koaxial umgebend angeordnet. Ferner ist ein Stellelement der Projektileinheit 18h das Ultraschallhorn umgebend angeordnet. Weiterhin ist ein Gegenlager der Projektileinheit 18h das Ultraschallhorn 16h umgebend angeordnet.

| | | | |
|---|---|---|---|
| 10 | Ultraschalleinheit | 64 | Proximaler Abschnitt |
| 12 | Sonotrode | 66 | Distaler Abschnitt |
| 14 | Ultraschallgenerator | 68 | Piezoaktor |
| 16 | Ultraschallhorn | 70 | distaler Endabschnitt |
| 18 | Projektileinheit | 72 | Mittelabschnitt |
| 20 | Projektil | 74 | Proximaler Endabschnitt |
| 22 | Führungskanal | 76 | Verjüngung |
| 26 | Führungsabschnitt | 78 | Distale Richtung |
| 28 | Fluidkanal | 80 | Ausnehmung |
| 30 | Fluiddurchlass | 82 | Innendurchmesser |
| 34 | Stellelement | 84 | Außendurchmesser |
| 36 | Feder | 86 | Außendurchmesser |
| 38 | Fixierelement | 88 | Stufe |
| 40 | Magnet | 90 | Außendurchmesser |
| 42 | Lithotripter | 92 | Außendurchmesser |
| 44 | Lithotripsiesystem | 94 | Verbindungseinheit |
| 46 | Steuergerät | 96 | Verbindungselement |
| 48 | Steuereinheit | 98 | Gewinde |
| 50 | Betätigungsmittel | 100 | Fluideinheit |
| 52 | Fluidpumpe | 102 | Fluidanschluss |
| 58 | Ultraschallaktor | 104 | Gegenlager |
| 60 | Gehäuse | 106 | Verfahrensschritt |
| 62 | Handhabe | 108 | Verfahrensschritt |
| 110 | | | Abdichtelement |

## Patentansprüche

1. Lithotripsievorrichtung, insbesondere intrakorporale Lithotripsievorrichtung, mit wenigstens einer Ultraschalleinheit (10a;10b), welche wenigstens eine Sonotrode (12a;12b), wenigstens einen Ultraschallgenerator (14a), der dazu ausgebildet ist Ultraschallwellen bereitzustellen, und wenigstens ein Ultraschallhorn (16a;16b; 16h), das zur Fokussierung und zumindest mittelbaren Übertragung der Ultraschallwellen von dem Ultraschallgenerator (14a) auf die Sonotrode (12a;112b) ausgebildet ist, umfasst, und mit wenigstens einer Projektileinheit (18a-h), welche wenigstens ein linear beweglich gelagertes Projektil (20a;20b;20c;e;g) umfasst, das dazu ausgebildet ist, in wenigstens einem Betriebszustand die Sonotrode (12a;12b) zumindest mittelbar zu beaufschlagen, und das in dem Betriebszustand zumindest mittelbar von dem Ultraschallgenerator (14a) mittels bereitgestellter Ultraschallwellen zu einer Bewegung angeregt wird, **dadurch gekennzeichnet, dass** das Projektil (20a;20b; 20c; 20e; 20g) im Bereich des Ultraschallhorns (16a;16b;16h) beweglich gelagert angeordnet ist, wobei das Projektil (20a;20b; 20c; 20e; 20g) das Ultraschallhorn (16a;16b;16h) koaxial umgebend oder innerhalb eines von dem Ultraschallhorn (16a;16b;16h) eingeschlossenen Volumens angeordnet ist.

2. Lithotripsievorrichtung nach Anspruch 1, **dadurch gekennzeichnet, dass** die Projektileinheit (18a-h) wenigstens einen Führungskanal (22a;g) umfasst, welcher zumindest zur beweglichen Lagerung des Projektils (20a; 20b; 20c; 20e; 20g; 20h) ausgebildet ist.

3. Lithotripsievorrichtung nach Anspruch 2, **dadurch gekennzeichnet, dass** die Ultraschalleinheit (10a;20b) zumindest teilweise den Führungskanal (22a;22g) ausbildet.

4. Lithotripsievorrichtung nach Anspruch 3, **dadurch gekennzeichnet, dass** das Ultraschallhorn (16a;16b;16h) wenigstens einen Führungsabschnitt (26a) aufweist, welcher zumindest teilweise den Führungskanal (22a;g) ausbildet.

5. Lithotripsievorrichtung nach einem der Ansprüche 2 bis 4, **dadurch gekennzeichnet, dass** der Führungskanal (22a; 22g) zumindest teilweise einen zu einer Fluidleitung ausgebildeten Fluidkanal (28a;28d;28g) ausbildet.

6. Lithotripsievorrichtung nach einem der vorhergehenden Ansprüche, **dadurch gekennzeichnet, dass** das Projektil (20a; 20b; 20c; 20e; 20g; 20h) wenigstens einen Fluiddurchlass (30a) aufweist.

7. Lithotripsievorrichtung nach einem der vorhergehenden Ansprüche, **dadurch gekennzeichnet, dass** das Projektil (20a; 20b; 20c; 20e; 20g; 20h) in dem Betriebszustand zumindest mittelbar über das Ultraschallhorn (16a;16b; 16h) von dem Ultraschallgenerator (14a) zu einer Bewegung angeregt wird.

8. Lithotripsievorrichtung nach einem der vorhergehenden Ansprüche, **dadurch gekennzeichnet, dass** das Projektil (20a; 20b; 20c; 20e; 20g, 20h) in einer Ausgangslage zumindest mittelbar an der Sonotrode (12a;12b) anliegt.

9. Lithotripsievorrichtung nach einem der vorhergehenden Ansprüche, **dadurch gekennzeichnet, dass** die Projektileinheit (18a-h) zumindest ein Stellelement (34a; 34d; 34e; 34f; 34g) umfasst, welches dazu ausgebildet ist, das Projektil (20a; 20b; 20c; 20e; 20g; 20h) in eine Ausgangslage rückzuführen.

10. Lithotripsievorrichtung nach den Ansprüchen 2 und 9, **dadurch gekennzeichnet, dass** das Stellelement (34a; 34d; 34e; 34f; 34g) in dem Führungskanal (22a;22g) zumindest teilweise angeordnet ist

11. Lithotripsievorrichtung nach Anspruch 9 oder 10, **dadurch gekennzeichnet, dass** das Stellelement (34a; 34d; 34e; 34f; 34g) als eine Feder (36a) ausgebildet ist.

12. Lithotripsievorrichtung nach einem der vorhergehenden Ansprüche, **dadurch gekennzeichnet, dass** die Projektileinheit (18a-h) zumindest ein Fixierelement (38b; 38c) umfasst, welches dazu ausgebildet ist, in wenigstens einem weiteren Betriebszustand das Projektil (20a; 20b; 20c; 20e; 20g; 20h) in einer Ausgangslage zu fixieren.

13. Lithotripsievorrichtung nach den Ansprüchen 2 und 12, **dadurch gekennzeichnet, dass** das Fixierelement (38b; 38c) außerhalb des Führungskanals (22a; 22g) angeordnet ist.

14. Lithotripsievorrichtung nach Anspruch 12 oder 13, **dadurch gekennzeichnet, dass** das Fixierelement (38b; 38c) als ein Magnet (40b) ausgebildet ist.

15. Lithotripsievorrichtung nach einem der vorhergehenden Ansprüche, **dadurch gekennzeichnet, dass** die Projektileinheit (18a-h) zur Einstellung einer Schlagleistung einer Bewegung des Projektils (20 a; 20b; 20c; 20e; 20g; 20h) ausgebildet ist.

16. Lithotripter (42a), insbesondere intrakorporaler Lithotripter, mit wenigstens einer Lithotripsievorrichtung nach einem der vorhergehenden Ansprüche.

17. Verfahren zum extrakorporalen Testbetrieb einer Lithotripsievorrichtung nach einem der Ansprüche 1 bis 15.

## Claims

1. A lithotripsy device, in particular an intracorporeal lithotripsy device, having at least one ultrasonic unit (10a; 10b), which has at least one sonotrode (12a; 12b), at least one ultrasonic generator (14a), which is designed to provide ultrasonic waves, and at least one ultrasonic horn (16a; 16b; 16h), which is designed for focusing and at least indirectly transmitting the ultrasonic waves from the ultrasonic generator (14a) to the sonotrode (12a; 112b), and having at least one projectile unit (18a-h), which comprises at least one projectile (20a; 20b; 20c;e;g) mounted for linear movement, which is designed to at least indirectly act on the sonotrode (12a; 12b) in at least one operating state, and which, in the operating state, is at least indirectly stimulated to move by the ultrasonic generator (14a) by means of ultrasonic waves provided, **characterized in that** the projectile (20a; 20b; 20c; 20e; 20g) is movably mounted in the area of the ultrasonic horn (16a; 16b; 16h), wherein the projectile (20a; 20b; 20c; 20e; 20g) is arranged coaxially surrounding the ultrasonic horn (16a; 16b; 16h) or within a volume enclosed by the ultrasonic horn (16a; 16b; 16h).

2. The lithotripsy device according to claim 1, **characterized in that** the projectile unit (18a-h) comprises at least one guide channel (22a; g), which is designed at least for the movable mounting of the projectile (20a; 20b; 20c; 20e; 20g; 20h).

3. The lithotripsy device according to claim 2, **characterized in that** the ultrasonic unit (10a; 20b) at least partially forms the guide channel (22a; 22g).

4. The lithotripsy device according to claim 3, **characterized in that** the ultrasonic horn (16a; 16b; 16h) has at least one guide section (26a), which at least partially forms the guide channel (22a; g).

5. The lithotripsy device according to any one of claims 2 to 4, **characterized in that** the guide channel (22a; 22g) at least partially forms a fluid channel (28a; 28d; 28g) designed to form a fluid line.

6. The lithotripsy device according to any one of the preceding claims, **characterized in that** the projectile (20a; 20b; 20c; 20e; 20g; 20h) has at least one fluid passage (30a).

7. The lithotripsy device according to any one of the preceding claims, **characterized in that** the projectile (20a; 20b; 20c; 20e; 20g; 20h) is stimulated to move in the operating state at least indirectly via the ultrasonic horn (16a; 16b; 16h) by the ultrasonic generator (14a).

8. The lithotripsy device according to any one of the preceding claims, **characterized in that** the projectile (20a; 20b; 20c; 20e; 20g, 20h) rests at least indirectly on the sonotrode (12a; 12b) in an initial position.

9. The lithotripsy device according to any one of the preceding claims, **characterized in that** the projectile unit (18a-h) comprises at least one adjusting element (34a; 34d; 34e; 34f; 34g), which is designed to return the projectile (20a; 20b; 20c; 20e; 20g; 20h) to a starting position.

10. The lithotripsy device according to claims 2 and 9, **characterized in that** the adjusting element (34a; 34d; 34e; 34f; 34g) is at least partially arranged in the guide channel (22a; 22g).

11. The lithotripsy device according to claim 9 or 10, **characterized in that** the adjusting element (34a; 34d; 34e; 34f; 34g) is designed as a spring (36a).

12. The lithotripsy device according to any one of the preceding claims, **characterized in that** the projectile unit (18a-h) comprises at least one fixing element (38b; 38c), which is designed to fix the projectile (20a; 20b; 20c; 20e; 20g; 20h) in an initial position in at least one further operating state.

13. The lithotripsy device according to claims 2 and 12, **characterized in that** the fixing element (38b; 38c) is arranged outside the guide channel (22a; 22g).

14. The lithotripsy device according to claim 12 or 13, **characterized in that** the fixing element (38b; 38c) is designed as a magnet (40b).

15. The lithotripsy device according to any one of the preceding claims, **characterized in that** the projectile unit (18a-h) is designed to adjust an impact power of a movement of the projectile (20a; 20b; 20c; 20e; 20g; 20h).

16. A lithotripter (42a), in particular an intracorporeal lithotripter, having at least one lithotripsy device according to any one of the preceding claims.

17. A method for the extracorporeal test operation of a lithotripsy device according to any one of claims 1 to 15.

## Revendications

1. Dispositif de lithotripsie, en particulier dispositif de lithotripsie intracorporelle, comportant au moins une unité à ultrasons (10a ; 10b) qui comprend au moins une sonotrode (12a ; 12b), au moins un générateur d'ultrasons (14a) conçu pour fournir des ondes ultrasonores, et au moins une corne à ultrasons (16a ; 16b ; 16h ) qui est conçue pour focaliser et transmettre au moins indirectement les ondes ultrasonores du générateur d'ultrasons (14a) à la sonotrode (12a ; 112b), et comportant au moins une unité de projectile ( 18a-h), qui comprend au moins un projectile (20a ; 20b ; 20c ; e ; g) monté mobile linéairement, qui est conçu pour agir au moins indirectement sur la sonotrode (12a ; 12b) dans au moins un état de fonctionnement, et qui dans l'état de fonctionnement, est excité au moins indirectement par le générateur d'ultrasons (14a) pour un mouvement au moyen d'ondes ultrasonores fournies, **caractérisé en ce que** le projectile (20a ; 20b ; 20c ; 20e ; 20g) est agencé monté mobile dans la zone de la corne à ultrasons (16a ; 16b ; 16h), dans lequel le
projectile (20a ; 20b ; 20c ; 20e ; 20g) est agencé coaxialement autour de la corne à ultrasons (16a ; 16b ; 16h) ou à l'intérieur d'un volume entouré par la corne à ultrasons (16a ; 16b ; 16h).

2. Dispositif de lithotripsie selon la revendication 1, **caractérisé en ce que** l'unité de projectile (18a-h) comprend au moins un canal de guidage (22a ; g) qui est conçu au moins pour le montage mobile du projectile (20a ; 20b ; 20c ; 20e ; 20g ; 20h).

3. Dispositif de lithotripsie selon la revendication 2, **caractérisé en ce que** l'unité à ultrasons (10a ; 20b) forme au moins partiellement le canal de guidage (22a ; 22g).

4. Dispositif de lithotripsie selon la revendication 3, **caractérisé en ce que** la corne à ultrasons (16a ; 16b ; 16h) présente au moins une section de guidage (26a) qui forme au moins partiellement le canal de guidage (22a ; g).

5. Dispositif de lithotripsie selon l'une des revendications 2 à 4, **caractérisé en ce que** le canal de guidage (22a ; 22g) forme au moins partiellement un canal de fluide (28a ; 28d ; 28g) conçu sous forme d'une conduite de fluide.

6. Dispositif de lithotripsie selon l'une des revendications précédentes, **caractérisé en ce que** le projectile (20a ; 20b ; 20c ; 20e ; 20g ; 20h) présente au moins un passage de fluide (30a).

7. Dispositif de lithotripsie selon l'une des revendications précédentes, **caractérisé en ce que** le projectile (20a ; 20b ; 20c ; 20e ; 20g ; 20h) est excité pour se déplacer dans l'état de fonctionnement au moins indirectement par l'intermédiaire de la corne à ultrasons (16a ; 16b ; 16h) par le générateur d'ultrasons (14a).

8. Dispositif de lithotripsie selon l'une des revendications précédentes, **caractérisé en ce que** le projectile (20a ; 20b ; 20c ; 20e ; 20g, 20h) repose au moins indirectement sur la sonotrode (12a ; 12b) dans une position initiale.

9. Dispositif de lithotripsie selon l'une des revendications précédentes, **caractérisé en ce que** l'unité de projectile (18a-h) comprend au moins un élément de réglage (34a ; 34d ; 34e ; 34f ; 34g) qui est conçu pour ramener le projectile (20a ; 20b ; 20c ; 20e ; 20g ; 20h) dans une position initiale.

10. Dispositif de lithotripsie selon les revendications 2 et 9, **caractérisé en ce que** l'élément de réglage (34a ; 34d ; 34e ; 34f ; 34g) est agencé au moins partiellement dans le canal de guidage (22a ; 22g).

11. Dispositif de lithotripsie selon la revendication 9 ou 10, **caractérisé en ce que** l'élément de réglage (34a ; 34d ; 34e ; 34f ; 34g) est conçu sous forme de ressort (36a).

12. Dispositif de lithotripsie selon l'une des revendications précédentes, **caractérisé en ce que** l'unité de projectile (18a-h) comprend au moins un élément de fixation (38b ; 38c) qui est conçu pour fixer le projectile (20a ; 20b ; 20c ; 20e ; 20g ; 20h) dans une position initiale dans au moins un autre état de fonctionnement.

13. Dispositif de lithotripsie selon les revendications 2 et 12, **caractérisé en ce que** l'élément de fixation (38b ; 38c) est agencé à l'extérieur du canal de guidage (22a ; 22g).

14. Dispositif de lithotripsie selon la revendication 12 ou 13, **caractérisé en ce que** l'élément de fixation (38b ; 38c) est conçu sous la forme d'un aimant (40b).

15. Dispositif de lithotripsie selon l'une des revendications précédentes, **caractérisé en ce que** l'unité de projectile (18a-h) est conçue pour ajuster une puissance d'impact d'un mouvement du projectile (20a ; 20b ; 20c ; 20e ; 20g ; 20h).

16. Lithotripteur (42a), notamment lithotripteur intracorporel, comportant au moins un dispositif de lithotripsie selon l'une des revendications précédentes.

17. Procédé pour le fonctionnement de test extracorporel d'un dispositif de lithotripsie selon l'une des revendications 1 à 15.
